# EUROPEAN PATENT APPLICATION

(11) **EP 3 610 880 A1**
(43) Date of publication of application: **19.02.2020**
(21) Application number: 18770912.6
(22) Date of filing: 22.03.2018
(51) Int. Cl.: A61K 31/7125, A61K 47/36, A61P 35/00, C12N 5/09

(54) **CANCER CELL ADHESION ACTIVITY INHIBITOR**

(30) Priority: 23.03.2017 JP 2017057975
(71) Applicant: NapaJen Pharma, Inc., Burlingame, California 94010 (US)
(72) Inventor: HIGUCHI, Sadaharu, Koganei-shi Tokyo 184-0012 (JP); AMANO, Kanako, Koganei-shi Tokyo 184-0012 (JP); USHIDA, Maki, Koganei-shi Tokyo 184-0012 (JP); ANDO, Hironori, Koganei-shi Tokyo 184-0012 (JP)
(74) Representative: Müller-Boré & Partner Patentanwälte PartG mbB
(86) International application number: PCT/JP2018/011330
(87) International publication number: WO 2018/174140

(57) **Abstract**

The purpose of the present invention is to provide a drug that is capable of effectively inhibiting adhesion activity, growth, and engraftment of cancer cells, using an oligodeoxynucleotide (ODN) in which phosphodiester bonds are phosphorothioated (S-configured). Adhesion activity, growth inhibitory capacity, and engraftment inhibitory capacity of cancer cells are rapidly improved as a result of S-configured ODN being formed into a composite with a polysaccharide having a β-1,3-glucan backbone.

## Description

### TECHNICAL FIELD

The present invention relates to an agent for inhibiting adhesion activity of cancer cells including a nucleic acid-polysaccharide complex. The present invention further relates to an agent for inhibiting proliferation of cancer cells, an agent for inhibiting engraftment of cancer cells, an agent for inhibiting cancer metastasis, and an agent for treating cancer respectively including a nucleic acid-polysaccharide complex.

### BACKGROUND ART

The nucleic acid-polysaccharide complexes" are "polymer complexes" in which "a functional nucleic acid molecule bonded to an end of polydeoxyadenine (polydA)" and "β-1,3-glucan" are complexed through hydrogen bonding and hydrophobic interaction. The nucleic acid-polysaccharide complexes are known to exert pharmacological effects required of functional nucleic acids included in these complexes when introduced into cells specifically targeted by Dectin-1 receptors (Patent Documents 1 and 2).

In the meantime, CpG oligodeoxynucleotides (CpG ODNs) are known to stimulate TLR9 (toll-like receptor 9) and activate natural immunity, and thus have been developed into adjuvant products for use in infection vaccines against foreign viral antigens. In the meantime, cancer therapeutic drugs using this activated natural immunity were developed until around 2009.

Recently, complexes between B/K-type CpG ODNs and schizophyllan (SPG) were found to possess powerful immunostimulating actions and anticancer activities (Patent Document 3). Some papers reveal a possible finding that phosphorothioated CpG ODNs (S-CpG ODNs) nonspecifically inhibits cell adhesion and induces apoptosis (Non-patent Document 1). The effects of CpG ODNs that have so far been reported, however, all rely on immunoreactivity. Thus, any technology is yet to be known that can significantly improve CpG ODN-assisted adhesion inhibitory actions without having to rely on immunoreactivity.

### PRIOR ART DOCUMENTS

### NON-PATENT DOCUMENTS

Non-patent Document 1: Eitan Okun et al., Journal of Neuroscience Research 87:1947-1952 (2009)

### PATENT DOCUMENTS

Patent Document 1: WO 2001-34207 A
Patent Document 2: WO 2004-100965 A
Patent Document 3: WO 2016-103531 A

### SUMMARY OF THE INVENTION

### PROBLEMS TO BE SOLVED BY THE INVENTION

The present invention is directed to providing drugs using phosphorothioated oligodeoxynucleotides (S-ODNs) that can effectively inhibit the adhesion activity, growth, and engraftment of cancer cells. The present invention is also directed to providing drugs using phosphorothioated oligodeoxynucleotides (S-ODNs) that are efficacious for cancer treatments and against cancer metastasis.

### MEANS FOR SOLVING THE PROBLEM

The inventors of this invention were committed to finding solutions to the known problems, and they were led to the following finding; performances of inhibiting the adhesion activity, growth, and engraftment of cancer cells are exponentially improvable by combining the S-ODN with a polysaccharide having a β-1,3-glucan skeleton into a complex (S-ODN/BG complex). Another finding is that the S-ODN/BG complex does not achieve the effect described above solely when the ODN used is CpG ODN, and such an activity of the S-ODN/BG complex is exhibited in an ODN sequence-nonspecific manner. The S-ODN/BG complex was found to inhibit the engraftment of cancer cells when the CpG ODNs were used as ODN, however, presented no sign of activated natural immunity as anticipated. Thus, the activities of S-ODN/BG complex using S-CpG ODN, which include the inhibition of cancer cell engraftment, do not result from the known CpG ODN immunoreactivity but attribute to a mechanism(s) of action yet to be reported. The present invention was accomplished through further discussions and experiments based on these findings.

The present invention provides drugs according to the following aspects. Aspect 1-1.

An agent for inhibiting adhesion activity of cancer cells including a complex of nucleic acid molecules and a polysaccharide, wherein the nucleic acid molecule includes an oligodeoxynucleotide in which phosphodiester bonds are phosphorothioated, and the polysaccharide is a polysaccharide having a β-1,3-glucan skeleton.

### Aspect 1-2.

An agent for inhibiting proliferation of cancer cells including a complex of nucleic acid molecules and a polysaccharide, wherein the nucleic acid molecule includes an oligodeoxynucleotide in which phosphodiester bonds are phosphorothioated, and the polysaccharide is a polysaccharide having a β-1,3-glucan skeleton.

### Aspect 1-3.

An agent for inhibiting engraftment of cancer cells including a complex of nucleic acid molecules and a polysaccharide, wherein the nucleic acid molecule includes an oligodeoxynucleotide in which phosphodiester bonds are phosphorothioated, and the polysaccharide is a polysaccharide having a β-1,3-glucan skeleton.

### Aspect 1-4.

An agent for inhibiting cancer metastasis including a complex of nucleic acid molecules and a polysaccharide, wherein the nucleic acid molecule includes an oligodeoxynucleotide in which phosphodiester bonds are phosphorothioated, and the polysaccharide is a polysaccharide having a β-1,3-glucan skeleton. Aspect 1-5.

An agent for treating cancer including a complex of nucleic acid molecules and a polysaccharide wherein the nucleic acid molecule includes an oligodeoxynucleotide in which phosphodiester bonds are phosphorothioated (except for CpG oligodeoxynucleotides), and the polysaccharide is a polysaccharide having a β-1,3-glucan skeleton.

### Aspect 1-6.

The agent according to any one of the aspects 1-1 to 1-5, wherein the polysaccharide is schizophyllan.

### Aspect 1-7.

The agent according to any one of the aspects 1-1 to 1-6, wherein polydeoxyadenine is added to the oligodeoxynucleotide.

### Aspect 1-8.

The agent according to the aspect 1-7, wherein the polydeoxyadenine includes 10 to 100 deoxyadenines.

### Aspect 1-9.

The agent according to any one of the aspects 1-1 to 1-8, wherein the oligodeoxynucleotide has 5 to 40 bases.

The present invention provides methods according to the following aspects. Aspect 2-1.

A method for inhibiting adhesion activity of cancer cells, the method including a step of administering a complex of nucleic acid molecules and a polysaccharide to a person in whom the adhesion activity of cancer cells needs to be inhibited, wherein the nucleic acid molecule includes an oligodeoxynucleotide in which phosphodiester bonds are phosphorothioated, and the polysaccharide is a polysaccharide having a β-1,3-glucan skeleton.

### Aspect 2-2.

A method for inhibiting proliferation of cancer cells, the method including a step of administering a complex of nucleic acid molecules and a polysaccharide to a person in whom the growth of cancer cells needs to be inhibited, wherein the nucleic acid molecule includes an oligodeoxynucleotide in which phosphodiester bonds are phosphorothioated, and the polysaccharide is a polysaccharide having a β-1,3-glucan skeleton.

### Aspect 2-3.

A method for inhibiting engraftment of cancer cells, the method including a step of administering a complex of nucleic acid molecules and a polysaccharide to a person in whom the engraftment of cancer cells needs to be inhibited, wherein the nucleic acid molecule includes an oligodeoxynucleotide in which phosphodiester bonds are phosphorothioated, and the polysaccharide is a polysaccharide having a β-1,3-glucan skeleton.

### Aspect 2-4.

A method for inhibiting cancer metastasis, the method including a step of administering a complex of nucleic acid molecules and a polysaccharide to a person in whom the cancer metastasis needs to be inhibited, wherein the nucleic acid molecule includes an oligodeoxynucleotide in which phosphodiester bonds are phosphorothioated, and the polysaccharide is a polysaccharide having a β-1,3-glucan skeleton.

### Aspect 2-5.

A method for treating cencer including a step of administering a complex of nucleic acid molecules and a polysaccharide to a cancer patient, wherein the nucleic acid molecule includes an oligodeoxynucleotide in which phosphodiester bonds are phosphorothioated (except for CpG oligodeoxynucleotides), and the polysaccharide is a polysaccharide having a β-1,3-glucan skeleton.

### Aspect 2-6.

The method according to any one of the aspects 2-1 to 2-5, wherein the polysaccharide is schizophyllan.

### Aspect 2-7.

The method according to any one of the aspects 2-1 to 2-6, wherein polydeoxyadenine is added to the oligodeoxynucleotide.

### Aspect 2-8.

The method according to the aspect 2-7, wherein the polydeoxyadenine includes 10 to 100 deoxyadenines.

### Aspect 2-9.

The method according to any one of the aspects 2-1 to 2-8, wherein the oligodeoxynucleotide has 5 to 40 bases.

The present invention provides uses of a complex according to the following aspects.

### Aspect 3-1.

Use of a complex of nucleic acid molecules and a polysaccharide for the manufacture of an agent for inhibiting adhesion activity of cancer cells, wherein the nucleic acid molecule includes an oligodeoxynucleotide in which phosphodiester bonds are phosphorothioated, and the polysaccharide is a polysaccharide having a β-1,3-glucan skeleton.

### Aspect 3-2.

Use of a complex of nucleic acid molecules and a polysaccharide for the manufacture of an agent for inhibiting proliferation of cancer cells, wherein the nucleic acid molecule includes an oligodeoxynucleotide in which phosphodiester bonds are phosphorothioated, and the polysaccharide is a polysaccharide having a β-1,3-glucan skeleton.

### Aspect 3-3.

Use of a complex of nucleic acid molecules and a polysaccharide for the manufacture of an agent for inhibiting engraftment of cancer cells, wherein the nucleic acid molecule includes an oligodeoxynucleotide in which phosphodiester bonds are phosphorothioated, and the polysaccharide is a polysaccharide having a β-1,3-glucan skeleton.

### Aspect 3-4.

Use of a complex of nucleic acid molecules and a polysaccharide for the manufacture of an agent for inhibiting cancer metastasis, wherein the nucleic acid molecule includes an oligodeoxynucleotide in which phosphodiester bonds are phosphorothioated, and the polysaccharide is a polysaccharide having a β-1,3-glucan skeleton.

### Aspect 3-5.

Use of a complex of nucleic acid molecules and a polysaccharide for the manufacture of an agent for treating cancer, wherein the nucleic acid molecule includes an oligodeoxynucleotide in which phosphodiester bonds are phosphorothioated (except for CpG oligodeoxynucleotides), and the polysaccharide is a polysaccharide having a β-1,3-glucan skeleton.

### Aspect 3-6.

The use according to any one of the aspects 3-1 to 3-5, wherein the polysaccharide is schizophyllan.

### Aspect 3-7.

The use according to any one of the aspects 3-1 to 3-6, wherein polydeoxyadenine is added to the oligodeoxynucleotide.

### Aspect 3-8.

The use according to the aspect 3-7, wherein the polydeoxyadenine includes 10 to 100 deoxyadenines.

### Aspect 3-9.

The use according to any one of the aspects 3-1 to 3-8, wherein the oligodeoxynucleotide has 5 to 40 bases.

### ADVANTAGES EFFECTS OF THE INVENTION

According to the present invention, use of the S-ODN/BG complex provides significantly improved performances of inhibiting the adhesion activity, growth, and engraftment of cancer cells which are inherent characteristics of ODN. The inhibitory actions of the S-ODN/BG complex do not result from inactivation of immune functions mediated by, for example, activated T cells. These inhibitory actions attribute to a yet-to-be-known mechanism(s) of action of the S-ODN/BG complex.

### BRIEF DESCRIPTION OF THE DRAWINGS

Fig. 1 shows a microscopic observation result (test 3) of states of adhesion, to wells, of a mouse colorectal cancer cell line (luciferase-transduced colon26) which was cultured in the presence of S-ODNs (ODN#1, ODN#2, ODN#3) or S-ODN/BG complexes (ODN#1/SPG complex, ODN#2/SPG complex, ODN#3/SPG complex).
Fig. 2 shows an evaluation result (test 3) of numbers of cells adhered to wells in a mouse colorectal cancer cell line (luciferase-transduced colon26) which was cultured in the presence of S-ODNs (ODN#1, ODN#2, ODN#3) or S-ODN/BG complexes (ODN#1/SPG complex, ODN#2/SPG complex, ODN#3/SPG complex) and subjected to a luciferase assay.
Fig. 3 shows an evaluation result (test 4) of adhesion activity, to extracellular matrix proteins, of a mouse colorectal cancer cell line (luciferase-transduced colon26) which was cultured with use of an extracellular matrix-coated assay plate wp in the presence of an S-ODN (ODN#4) or an S-ODN/BG complex (ODN#4/SPG complex).
Fig. 4 shows a measurement result (test 5) of MHC class I expression levels in a mouse colorectal cancer cell line (luciferase-transduced colon26) which was cultured in the presence of an S-ODN (ODN#3) or S-ODN/BG complexes (ODN#2/SPG complex, ODN#3/SPG complex).
Fig. 5 shows an analysis result (test 6) of ratios between living cells and dead cells in a mouse colorectal cancer cell line (luciferase-transduced colon26) which was cultured in the presence of an S-ODN (ODN#3) or and S-ODN/BG complex (ODN#3/SPG complex).
Fig. 6 shows an evaluation result (test 9) of engraftment, into lungs, of mouse colorectal cancer cells (luciferase-transduced colon26) which was cultured in the presence of an S-ODN (OODN#3-dA(s)40) or an S-ODN/BG complex (ODN#3/SPG complex) in 5 days and 10 days after the cancer cells were administered to the mice.
Fig.7 shows an evaluation result (test 10) of engraftment, into lungs of mice, of mouse colorectal cancer cells (luciferase-transduced colon26) in 10 days after the cancer cells, an S-ODN (OODN#3-dA(s)40) or an S-ODN/BG complex (ODN#3/SPG complex) was administered to the mice.
Fig.8 shows an evaluation result (test 11) of engraftment, into lungs of mice, of mouse colorectal cancer cells (luciferase-transduced colon26) in 10 days after the cancer cells, an S-ODN (OODN#3-dA(s)40) or an S-ODN/BG complex (ODN#3/SPG complex) was administered to the mice.
Fig.9 shows an evaluation result (test 12) of engraftment, into lungs of mice, of mouse colorectal cancer cells (luciferase-transduced colon26) in 6 days after the cancer cells, an S-ODN (OODN#3-dA(s)40) or an S-ODN/BG complex (ODN#3/SPG complex) was administered to the mice.
Fig.10 shows an evaluation result (test 13) of engraftment, into lungs of mice, of mouse colorectal cancer cells (luciferase-transduced colon26) in 5 days after the cancer cells, an S-ODN (OODN#3-dA(s)40) or an S-ODN/BG complex (ODN#3/SPG complex) was administered to the mice.
Fig.11 shows an evaluation result (test 14) of engraftment, into lungs of mice, of mouse colorectal cancer cells (luciferase-transduced colon26) in 5 days after the cancer cells, an S-ODN (OODN#3-dA(s)40) or an S-ODN/BG complex (ODN#3/SPG complex) was administered to the mice.

### EMBODIMENTS OF THE INVENTION

An agent for inhibiting adhesion activity of cancer cells, an agent for inhibiting proliferation of cancer cells, an agent for inhibiting engraftment of cancer cells, an agent for inhibiting cancer metastasis, and an agent for treating cancer according to the present invention are hereinafter described in detail. The description given below of the agents such as the agent for inhibiting adhesion activity of cancer cells applies likewise to specific embodiments of a use and a method according to the present invention.

### S-ODN

The present invention uses, as nucleic acid molecules, ODNs in which phosphodiester bonds are phosphorothioated (S-ODN). According to the present invention, combined use of the S-ODN and SPG provides significantly improved performances of inhibiting the adhesion activity, growth, and engraftment of cancer cells which are inherent characteristics of the ODNs.

As demonstrated in tests described later, effects of the present invention are exerted nonspecifically to ODN base sequences. In the present invention, therefore, any ODNs may be used irrespective of their base sequences. The ODNs used in the present invention may be selected from the functionally known ODNs such as CpG ODN or may be selected from any functionally unknown ODNs having random base sequences. In this regard, however, the present invention uses any ODNs but CpG ODNs to prepare the cancer therapeutic drug.

The CpG ODNs are ODNs having CpG motifs including unmethylated cytosine-guanine sequences. While the number of CpG motifs in a CpG ODN is not particularly limited, the CpG ODN may have 1 to 20 CpG motifs or preferably 1 to 10 CpG motifs. There are four known types of CpG ODNs; K type, D type, C type and P type, depending on their base sequences (Vollmer, J. & Krieg, A.M. Advanced drug delivery reviews 61, 195-204, 2009). The CpG ODNs used in the present invention may be of any one of these types.

The number of bases in the ODNs used in the present invention, though not particularly limited, may be 5 to 40, preferably 10 to 30, or more preferably 15 to 25.

The present invention uses ODNs in which phosphodiester bonds are phosphorothioated (S-ODNs). Use of such S-ODNs is a key factor in achieving greatly improved performances of inhibiting the adhesion activity, growth, and engraftment of cancer cells.

In the present invention, the "phosphodiester bond being phosphorothioated" represents a bonding structure in which one of oxygen atoms of a phosphoric acid residue in a phosphodiester bond site has been substituted with a sulfur atom. In the ODNs used in the present invention, all of phosphodiester bonds may preferably be phosphorothioated. The phosphodiester bonds, however, may be unphosphorothioated in part unless it compromises the effects to be achieved by the present invention. The ODNs may be phosphorothioated by any suitable one of the known methods.

In case the formation of a complex with a polysaccharide having a β-1,3-glucan skeleton (BG) is not directly possible with the ODNs used in this invention, polydeoxynucleotide that can form a complex with BG may desirably be added to at least 5' ends or 3' ends of the ODN. A suitable example of the polydeoxynucleotide that can form a complex with BG is polydeoxyadenine (polydA). The polydA that forms a triple helical structure with BG may easily provide a complex with BG.

The polydA may be optionally bonded to the ODN in any suitable manner. For example, the polydA may be bonded to 5' ends of the ODN alone, the polydA may be bonded to 3' ends of the ODN alone, or the polydA may be bonded to 3' ends and 5' ends of the ODN. Preferably, the polydA may be bonded to 3' ends of the ODN alone.

The polydA may be bonded by phosphoric acid bonding, directly or through a linker, to a terminal deoxyribonucleotide of the ODN.

The number of deoxyadenines in the polydA is not particularly limited insofar a complex is formable with schizophyllan described later. For example, the polydA may preferably consist of 10 to 100, preferably 20 to 100 deoxyadenines, more preferably 20 to 80 deoxyadenines, or even more preferably 30 to 50 deoxyadenines.

In any polydeoxynucleotide that can form a complex with BG such as polydA, phosphodiester bonds may preferably be phosphorothioated. While all of phosphodiester bonds in the polydeoxynucleotide may preferably be phosphorothioated, the phosphodiester bonds may be unphosphorothioated in part unless it compromises the effects to be achieved by the present invention. The polydeoxynucleotide may be phosphorothioated by any suitable one of the known methods.

### BG

The polysaccharide having a β-1,3-glucan skeleton (BG) has a main chain in which glucoses are coupled by β1→3 glucoside bonding. There are known polysaccharides that differ in the ratio of side chain glucose residues to main chain glucose residues (hereinafter, simply referred to as side-chain ratio). It has so far been disclosed that two strands of BG are bonded to a single-stranded polydeoxynucleotide to form a triple helical structure. In order to facilitate the formation of a complex with S-ODN, the BG used in the present invention may suitably be a polysaccharide having a β-1,3-glucan skeleton with a relatively high side-chain ratio.

Examples of the BG used in the present invention may include schizophyllan, lentinan, curdlan, pachyman, gliforan and scleroglucan. Of these examples, schizophyllan and lentinan may be preferable, between which schizophyllan may be more preferable.

The SPG (schizophyllan) is producible according to, for example, the usually employed method described in (A.C.S.38 (1), 253 (1997); Carbohydrate Research, 89, 121-135 (1981)). The schizophyllan thus obtained may have a desired molecular weight by being ultrasonically treated.

Functional molecules may be bonded to the BG described herein to an extent that does not undermine the effects to be achieved by the present invention. The ratio of functional molecules, if bonded to the BG, may be 1 to 200, preferably 1 to 100, or more preferably 1 to 50 functional molecules per 100 side chains of the BG. Such bonding ratios of functional molecules are adjustable by controlling the amount of an oxidant, such as sodium periodate, added to branched glucose residues in the method of production described earlier.

It is not particularly limited which sites in the BG functional molecules the functional molecules should be bonded to or linkers serving to couple the functional molecules should be bonded to. Preferably, the functional molecules or linkers may be bonded through substitution with 1,2-diol sites of glucose having 1,6-glucopyranoside bonds branched from β-1,3-glucan main chains of the BG.

The molecular weight of the BG used in the present invention is not particularly limited and may suitably be set in accordance with a chain length in a region of the S-ODN involved in the formation of a complex with the BG. For example, the molecular weight of SPG may be typically 25,000 to 500,000 or preferably 25,000 to 250,000.

### S-ODN/BG complex

This invention uses S-ODN/BG complexes in which an S-ODN at least in part and two strands of BG form a triple helical structure. Use of such a complex may provide significantly improved performances of inhibiting the adhesion activity, growth, and engraftment of cancer cells which are inherent characteristics of ODN.

The S-ODN/BG complexes may be formed by any suitable one of the polydeoxynucleotide-BG complexing methods conventionally used.

An exemplified method for complexing SPG with polydA-added S-ODN (S-polydA-ODN) may include the following steps 1) to 3); 1) preparing the S-polydA-ODN using a known method, 2) separately preparing SPG or preparing SPG to which functional molecules are bonded directly or through linkers (modified SPG), and 3) forming a complex using single-stranded polydA sites included in the S-polydA-ODN and the SPG or modified SPG.

In the step 3) of the method, a mixing ratio between the S-polydA-ODN and the SPG or modified SPG may suitably be decided in accordance with a chain length in the SPG or modified SPG. One strand of the polydA and two strands of the SPG form a triple helical structure in which one glucose molecule in the main chain of the SPG corresponds to one adenine molecule of the polydA. In the SPG - S-polydA-ODN complex, the polydA is captured at one or two or more positions in a double helical structure consisting of two strands of SPG, which forms the triple helical structure. For example, an S-polydA-ODN containing 40-mer polydA and SPG having the molecular weight of 150,000 may form a triple helical structure that contains 17 molecules of the S-polydA-ODN for two molecules of the SPG having the molecular weight of 150,000. The S-polydA-ODN and the SPG may be mixed at the molar ratio of 20:1 to 1:5 or preferably at the molar ratio of 10:1 to 1:1, in which it is preferable to complex a single-stranded polydA site in the S-polydA-ODN with the SPG or modified SPG. By thus complexing the S-ODN and the SPG or modified SPG under predefined conditions, these materials may be efficiently interacted with each other, which allows the S-polydA-ODN - SPG complex to be more efficiently produced.

The triple helical structure in the S-polydA-ODN - SPG complex may be specifically achieved by the following method. The SPG has a triple helical structure in nature or in water. This SPG is dissolved in a polar solvent of, for example, DMSO (dimethyl sulfoxide) or in an alkali aqueous solution, for example, a sodium hydroxide aqueous solution and denatured into a single-stranded state, to which the S-polydA-ODN is then added. By reverting the solvent into water or by neutralizing the alkali aqueous solution (reproduction process), a complexed triple helical structure (associative structure) is formed that consists of two strands of SPG and a single-stranded site of the polydA included in the S-polydA-ODN. It is considered that the polydA and SPG are thus complexed mostly through hydrogen bonding and hydrophobic interaction.

### Applications of use

In the S-ODN/BG complex described so far, inherent characteristics of ODN; performances of inhibiting the adhesion activity, growth, and engraftment of cancer cells, are significantly improved. The S-ODN/BG complex, therefore, may be advantageously useful as an agent for inhibiting adhesion activity of cancer cells, an agent for inhibiting proliferation of cancer cells and/or an agent for inhibiting engraftment of cancer cells.

The "agent for inhibiting adhesion activity of cancer cells" described herein is a drug that inhibits cancer cells from adhering to healthy cells or healthy tissues. The "agent for inhibiting proliferation of cancer cells" is a drug that inhibits the growth of cancer cells and that prevents increase of or decreases the count of cancer cells. The "agent for inhibiting engraftment of cancer cells" described herein is a drug that inhibits cancer cells adhered to healthy cells or healthy tissues from functioning as cancerous tissues.

Types of cancers in which the adhesion activity, growth and/or engraftment of cancer cells are desirably inhibited, though not particularly limited, may include liver cancer, pancreatic cancer, lung cancer, breast cancer, bladder cancer, prostate cancer, cervical cancer, kidney cancer, esophageal cancer, colorectal cancer, colon cancer, stomach cancer, rectal cancer, brain tumor, head and neck cancers, bile duct cancer, gallbladder cancer, oral cavity cancer, skin cancer, uterine body cancer, solid cancer like thyroid cancer, and blood cancers including leukemia and malignant lymphoma.

When the S-ODN/BG complex is used as the agent for inhibiting adhesion activity of cancer cells, agent for inhibiting proliferation of cancer cells and/or agent for inhibiting engraftment of cancer cells, routes for administration are not particularly limited and may suitably be selected, in accordance with a patient's symptoms, condition and disease, from the known methods of administration, for example, oral administration, non-oral administrations (including intravenous, intra-abdominal, intramuscular, subcutaneous, endorectal and intravaginal administrations), inhalation, systematic administration, local administrations (including external applications to skin and cheek cavity and drip infusion into any site where substantially no invasion into bloodstream occurs such as eyes, ears and nose).

When the S-ODN/BG complex is used as the agent for inhibiting adhesion activity of cancer cells, agent for inhibiting proliferation of cancer cells and/or agent for inhibiting engraftment of cancer cells, doses to be administered are not particularly limited, because those skilled in the art should be able to decide effective and nontoxic doses of the S-ODN/BG complex so as to take desired drug effects based on routine tests. For example, possible doses of the S-ODN/BG complex may typically be selected from values of approximately 0.001 to 10 mg per 1 kg and per day.

A therapeutically effective amount of S-ODN/BG complex in combination with a pharmacologically acceptable carrier is formulated into a pharmaceutical product for use. Examples of such a carrier may be aqueous carriers including purified water, saccharide-containing aqueous solutions, buffer solutions, physiological salt solution and nuclease-free water, and excipients.

The inhibitions against the adhesion activity, growth, and engraftment of cancer cells should be effective for cancer treatments. The S-ODN/BG complex, therefore, may be useful as a cancer therapeutic drug. When the S-ODN/BG complex is used as a cancer therapeutic drug, the ODN constituting the S-ODN/BG complex is any ODNs but CpG ODNs. When the S-ODN/BG complex is used as an agent for treating cancer, types of targeted cancers, methods of administration, and doses are the same as those described earlier.

The adhesion activity, growth, and engraftment of cancer cells are closely associated with mechanisms of cancer metastasis. The S-ODN/BG complex, therefore, may be useful as an agent for inhibiting cancer metastasis. When the S-ODN/BG complex is used as the agent for inhibiting cancer metastasis, types of targeted cancers, methods of administration, and doses are the same as those described earlier.

### EXAMPLES

The present invention is hereinafter described in detail by way of examples. The examples should not be construed as imposing any limitation on the scope of the present invention.

### Test materials

### [S-polydA-ODNs and S-ODNs]

S-polydA-ODNs and S-ODNs were prepared that respectively had sequences shown in Table 1. In ODN#1-dA(S)40, ODN#2-dA(S)40, ODN#3-dA(s)40, ODN#4-dA(s)40, ODN#1, ODN#2 and ODN#3 shown in Table 1, all of phosphodiester bonds are phosphorothioated.

In the ODN#1-dA(S)40, 40-mer polydA is added to 3' ends of ODN #1; ODN with no CpG motif. In the ODN#2-dA(S)40, 40-mer polydA is added to 3' ends of ODN#2; ODN with no CpG motif, at three positions of which two or more guanines are coupled. In the ODN#3-dA(S)40, 40-mer polydA is added to 3' ends of ODN #3; CpG ODN with three CpG motifs. In the ODN#4-dA(S)40, 40-mer polydA is added to 3' ends of ODN#4; ODN with no CpG motif, at three positions of which two or more guanines are coupled.

**[Table 1]**

| S-polydA-ODN | Base sequence (5' -> 3') |
|---|---|
| ODN#1-dA (S) 40 | |
| ODN#2-dA (S) 40 | |
| ODN#3-dA (s) 40 | |
| ODN#4-dA (s) 40 | |
| | |

| S-ODN | Base sequence (5' ->3') |
|---|---|
| ODN#1 | agttccattctcccatagcca (SEQ ID NO. 5) |
| ODN#2 | tggctatgggagaatggaact (SEQ ID NO. 6) |
| ODN#3 | atcgactctcgagcgttctc (SEQ ID NO. 7) |
| ODN#4 | atggactctggagggttctc (SEQ ID NO. 8) |

### (SPG)

SPG having the molecular weight of 150,000 was prepared.

### Preparation of S-ODN/BG complexes

S-ODN/BG complexes used in the respective tests were prepared as follows. The SPG was added to a 0.25 N sodium hydroxide aqueous solution at the final concentration of 15 mg/ml. The resulting solution was stirred through vibration for one hour and then left at rest for one day at 4°C to be denatured. Solutions were prepared in which the S-polydA-ODNs of Table 1 were respectively dissolved in 330 mM sodium dihydrogen phosphate. The solutions thus prepared were added to the denatured SPG solution and neutralized, and then left at rest for 24 hours or more at 4°C. Each of the solutions was added to the denatured SPG solution, in which the amount of SPG was adjusted to 0.27 mol for 1 mol of the S-polydA-ODN.

Of the S-ODN/BG complexes, one obtained with the SPG and ODN#1-dA(S)40 is referred to as "ODN#1/SPG complex", one obtained with the SPG and ODN#2-dA(S)40 as "ODN#2/SPG complex", one obtained with the SPG and ODN#3-dA(S)40 as "ODN#3/SPG complex", and one obtained with the SPG and ODN#4-dA(S)40 as "ODN#4/SPG complex".

### Test 1: Evaluation on performance of inhibiting the adhesion activity of colorectal cancer cells (1)

This test evaluated the performance of the ODN#3/SPG complex in inhibiting the adhesion activity of a mouse colorectal cancer cell line.

First, an ODN#3-containing solution and an ODN#3/SPG complex-containing solution were prepared with use of 1 × PBS (ThermoFisher) at the concentration of 10 µM. Further, an SPG-containing solution was prepared with use of 1 × PBS (ThermoFisher) at the concentration of 0.3 µg/µl. Then, 120 µl of the prepared ODN#3 solution, ODN#3/SPG complex solution, or SPG solution was added to 3 ml of colon26-Luc cells (JCRB1496)(2 × 10⁵ cells/ml) suspended in a 10% FBS-containing RPMI 1640 (ThermoFisher) culture medium and mixed well by pipetting. Then, 1 ml of each mixture was put in each well of a 24-well plate to culture the cells. The test materials were microscopically observed and photographed at day 1 after the culturing started. The colon26-Luc (JCRB1496) is a luciferase-transduced mouse colorectal cancer cell line. This cell line is known to express luciferase intra vitam.

As a result of microscopic observation of cells adhered to the wells, numerous cells were found to adhere to the bottoms of the ODN#3-treated well, SPG-treated well, and untreated (control) well, among which the numbers of adhered cells were hardly different. The ODN#3/SPG complex-coated well, however, resulted in a decrease in the number of cells adhered to the bottom. This result demonstrates that the ODN#3/SPG complex can be expected to inhibit the adhesion of colorectal cancer cells.

### Test 2: Evaluation on performance of inhibiting the adhesion activity of a malignant melanoma cell line

This test evaluated the performance of the ODN#3/SPG complex in inhibiting the adhesion activity of a malignant melanoma cell line. First, the ODN#3 (final concentration: 1,000 nM), ODN#3/SPG complex (final concentration: 500 nM or 1,000 nM), or SPG (final concentration: 29.3 µg/ml) was added to each well of a 24-well plate, and a cell suspension (DMEM (FBS 10%)) containing 2 × 10⁵ cells/ml of B16 F0-Luc (mouse malignant melanoma cell line, JCRB1474) was further added to each well to culture the cells. The B16 F0-Luc is luciferase-transduced mouse malignant melanoma cell line. This cell line is known to express luciferase intra vitam. The test materials were microscopically observed at day 2 after the culturing started. Further, another test was conducted, as a control test, similarly to the manner described earlier, except that the ODN#3, ODN#3/SPG complex or SPG was not added to the wells.

It was confirmed by microscopic observation on the cells adhered to the wells that the ODN#3/SPG complex inhibited the adhesion of malignant melanoma cells and also inhibited the adhesion activity of cancer cells irrespective of types of cancers.

### Test 3: Evaluation on performance of inhibiting the adhesion activity of colorectal cancer cells (2)

This test evaluated the performances of the ODN#1/SPG complex, ODN#2/SPG complex and ODN#3/SPG complex in inhibiting the adhesion activity of mouse colorectal cancer cells. First, 100 µl of a cell suspension containing 2 × 10⁵ cells/ml of Colon26-Luc (colon-26; mouse cell line that expresses luciferase, JCRB1496) (DMEM (FBS10%) was added to each well of a 96-well plate, and the ODN#1, ODN#2, ODN#3, ODN#1/SPG complex, ODN#2/SPG complex, or ODN#3/SPG complex was further added to each well at the final concentration of 1 µM to culture the cells. The Colon26-Luc is a luciferase-transduced Colon26; mouse colorectal cancer cell line. This cell line is known to express luciferase intra vitam. The test materials were microscopically observed and then subjected to luciferase assay to measure the count of cells adhered to each well at day 1 after the culturing started. Further, another test was conducted, as a control test, similarly to the manner described earlier, except that the ODN#3, ODN#3/SPG complex or SPG was not added to the wells.

The count of cells adhered to each well was measured as described below. First, the culture supernatant was removed from each well. Then, a step of adding 100 µl of RPMI (serum-free) to each well and removing the supernatant from each well was performed twice. Then, the luciferase (Luc) level in each well was measured with use of ONE-Glo™ Luciferase Assay System.

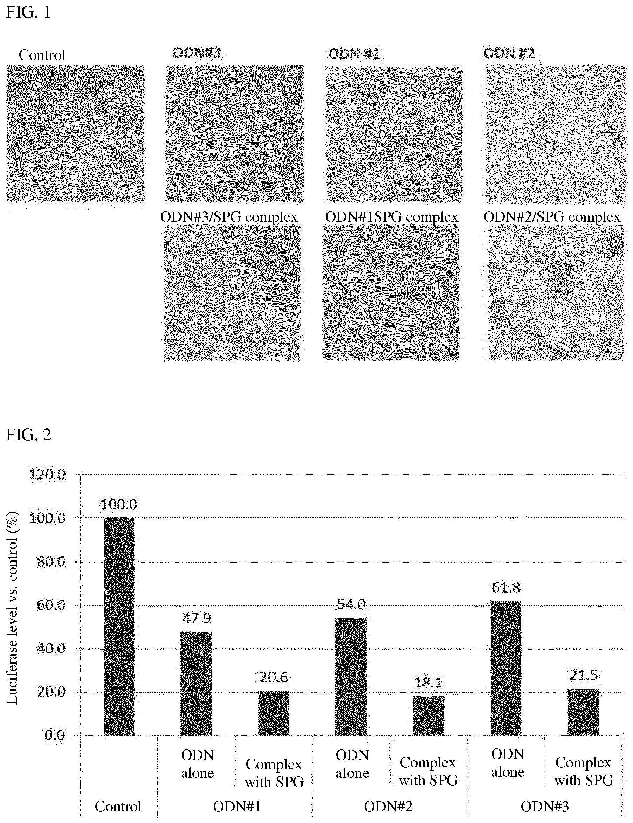

Fig. 1 shows a microscopic observation result of cells adhered to the wells. Fig. 2 shows a luciferase assay result. These results demonstrate that the ODN#1/SPG complex and ODN#2/SPG complex using the ODN with no CpG motif successfully inhibited the adhesion activity of colorectal cancer cells, similarly to the CpG ODN-used ODN#3/SPG complex.

### Test 4: Evaluation on performance of inhibiting the adhesion activity of colorectal cancer cells to extracellular matrixes

This test evaluated the performance of the ODN#4/SPG complex in inhibiting the adhesion activity of mouse colorectal cancer cells to extracellular matrixes.

Colon26-Luc cells (Colon-26; mouse cell line that expresses luciferase, JCRB1496) were cultured in 10% FBS-containing RPMI 1640. When the confluency was reached to 80%, the cells were detached from the culture dish and collected with use of Accutase (AT104, Innovative Cell Technologies, Inc.). The cells were cleaned in a RPMI 1640 (ThermoFisher) culture medium containing 0.5% BSA, 2mM CaCl₂ and 2mM MgCl₂. Then, the cells were suspended again in a similar RPMI 1640 culture medium to prepare a cell suspension of 1.0 × 10⁶ cells/ml.

The cell suspension was dispensed into a 1.5-ml tube, and 1.0 µM in total of the ODN#4/SPG complex and ODN#4 was added to the cell suspension and mixed well by pipetting. The resulting suspension was dispensed into wells; 150 µl/well, of the assay plate of the CytoSelect 48-well Cell Adhesion Assay Kit (CBA-071, CELL BIOLABS, INC.) that had been warmed for 10 minutes at room temperature to culture the cells for 90 minutes at 37°C in a 5%CO₂ atmosphere. The assay plate of the CytoSelect 48-well Cell Adhesion Assay Kit was evenly coated with extracellular matrix proteins (fibronectin, collagen I/IV, laminin, fibrinogen) and positive control protein (BSA) and is thus allowed to evaluate the adhesion activity to the extracellular matrix proteins.

The supernatant was removed after the culturing ended. Then, 150 µl of PBS containing 2 mM CaCl₂ and 2 mM MgCl₂ was dispensed into each well, and the PBS was then removed. This cleaning operation was performed four times. Additionally, a CyQuant GR dye diluted solution was prepared, in which a CyQuant GR dye attached to the CyQuant Select Assay Kit was diluted to 1:300 with a Lysis buffer to prepare a CyQuant GR dye-diluted solution. The PBS was removed from each well after the cleaning operation was completed, and 200 µl of the CyQuant GR dye-diluted solution was dispensed into each well. The wells were then shaken for 20 minutes at room temperature. From each well, 150 µl of the solution was transferred into a 96-well black plate to measure the intensity of fluorescence using a fluorescence plate reader (excitation 480 nm/fluorescence 520 nm). A greater fluorescence intensity value indicates that more cells are adhered to the well. Further, another test was conducted, as a control test, similarly to the manner described earlier, except that PBS was added instead of the ODN#4/SPG complex and ODN#4.

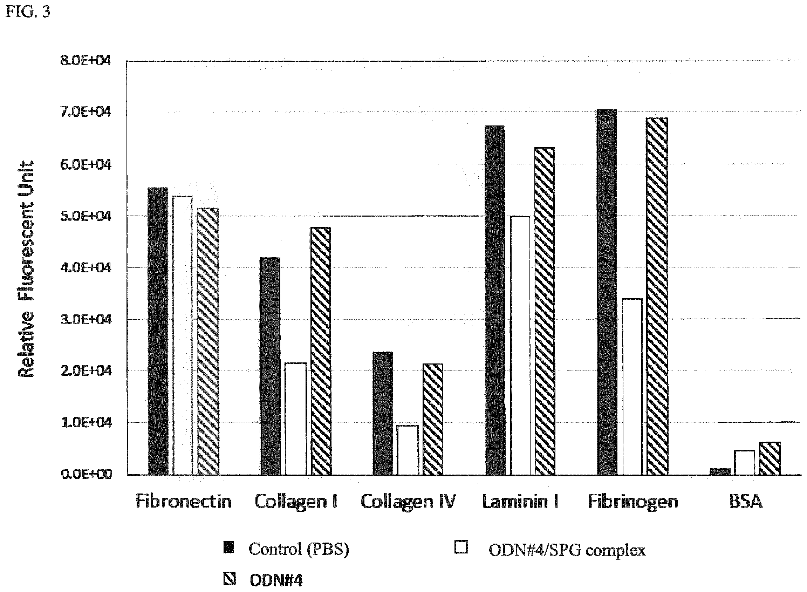

Fig. 3 shows the obtained result. The result showed no difference between the intensities of fluorescence of the ODN#4-treated colorectal cancer cells and untreated colorectal cancer cells. On the contrary, the ODN#4/SPG complex-treated colorectal cancer cells resulted in lower fluorescence intensities for all of the extracellular matrixes except for fibronectin. This teaches that the ODN#4/SPG complex selectively inhibits the adhesion of tumor cells to different extracellular matrixes. There was no sign of inhibiting the adhesive activity to the positive control; BSA, which confirmed that this test was properly carried out.

### Test 5: Analysis of impacts affecting the expression of MHC class I in cancer cells

This test evaluated impacts of the ODN#2/SPG complex and ODN#3/SPG complex affecting the expression of MHC class I in colorectal cancer cells. Colon26-Luc (Colon-26; mouse cell line that expresses luciferase, JCRB1496) were cultured in a 10-cm dish using RPMI (FBS 10%). When the confluency of 90% was reached, the colon26-Luc cells were detached from the dish and dispersed with use of Accutase (AT104, Innovative Cell Technologies, Inc.) and then collected. The collected colon26-Luc cells were cleaned and then suspended in RPMI (FBS 10%). The cells were then disseminated in T25 flasks at the rate of 3.0 × 10⁶ cells/flask. The amount of a culture medium used then was 4 mL/flask. To obtain the concentration of 500 nM, along with the colon26-Luc dissemination, the ODN#3, ODN#2/SPG complex, or ODN#3/SPG complex was added to each flask at the final concentration of 1 µM, and the cells were cultured for 20 hours at 37°C in a 5% CO₂ atmosphere. After the culturing was over, the colon26-Luc cells were detached from the T25 flasks and dispersed with Accutase (Innovative Cell Technologies) and then collected. The collected cells were cleaned and then suspended in an FCM buffer. Then, the count of cells was measured with the Cellometer (Nexcerom Bioscience). After the count of cells was measured, the cells were dyed using an anti-mouse MHC Class I antibody (H-2Kd) for FCM according to a usually employed method. Then, the dyed cells were mixed with a nuclear staining solution (7-AAD;7-Amino-Actinomycin D) and then analyzed by flow cytometry (Gallios, Beckman Coulter). Further, another test was conducted, as a control test, similarly to the manner described earlier, except that the ODN#3, ODN#2/SPG complex or ODN#3/SPG was not added.

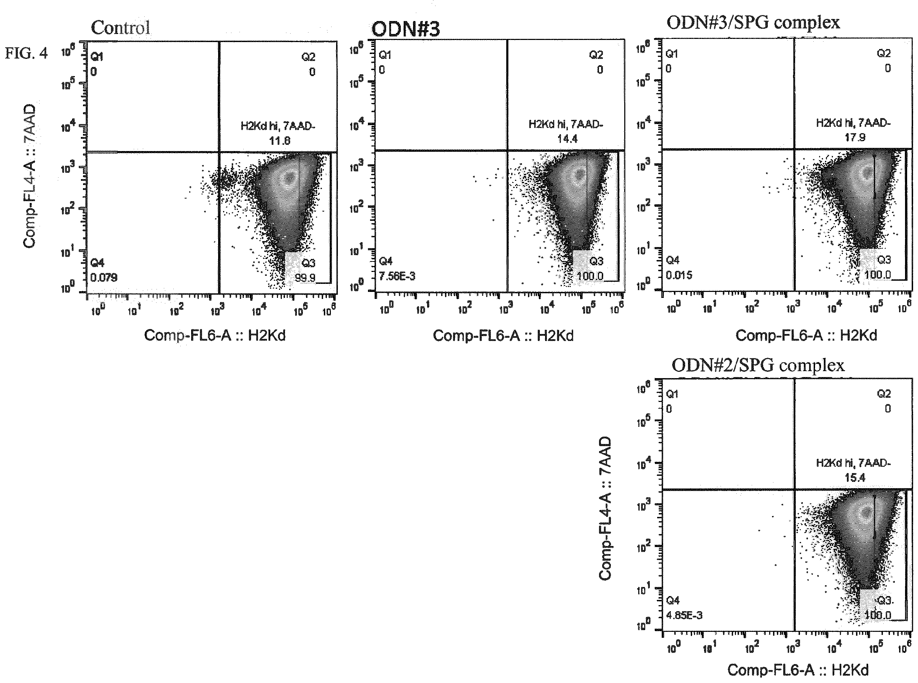

Fig. 4 shows the obtained result. According to this result, there were certain increases in cell colonies with high MHC class I expression levels when the ODN#3, ODN#2/SPG complex or ODN#3/SPG complex was added. In comparison between the ODN (ODN#3) and SPG complexes (ODN#2/SPG complex, ODN#3/SPG complex), cell colonies with high MHC class I expression levels further increased with the complexes than the ODN.

### Test 6: Evaluation on performance of inhibiting the growth of cancer cells

This test evaluated impacts of the ODN#3/SPG complex affecting the growth of colorectal cancer cells. First, 2 ml of a cell suspension containing 1 × 10⁶ cells/ml of Colon26-Luc (Colon-26; mouse cell line that expresses luciferase, JCRB1496) (RPMI (FBS10%)) was added to each well of a 6-well plate, and the ODN#3 or ODN#3/SPG complex was further added to each well at the final concentration of 400 µM to culture the cells. The Colon26-Luc is a luciferase-transduced Colon26; mouse colorectal cancer cell line. This cell line is known to express luciferase intra vitam. The culture supernatant was collected into a 15-ml tube in 2.5 hours and 48 hours after the culturing started. Further, 1 ml of PBS was added to each well from which the culture supernatant had been collected, and the wells were gently shaken. The content of each well was then collected into the same tube. Next, 0.5 ml of Trypsin EDTA was added to each well and subjected to a treatment at 37°C for 3 minutes to detach the cells from the wells. Then, PBS was added to each well, and the resulting content was collected into the same tube. The supernatant was centrifugally removed from the tube (400 g × 5 min.), and the cells collected then were suspended in 500 ml of PBS. Then, 1.5 ml of the obtained cell suspension was transferred into a new tube. The tube was cleaned with PBS, during which the amount of the solution was adjusted to approximately 1.5 ml. The supernatant was centrifugally removed from the tube (400 g × 5 min.), and the cells collected then were suspended in 250 µl of the Annexin V Binding Buffer (BioLegend). Then, 100µl of the obtained cell suspension was put in a 1.5-ml tube, and 5µl of the Annexine V-FITC (BioLegend) and 5µl of a nuclear staining solution (7-AAD or PI (Propidium Iodide)) were added to the tube and stirred with vortex under moderate conditions. The tube was then left at rest for 15 minutes at room temperature in a dark place. Next, 400µl of the Annexine V Binding Buffer was added to the tube, and the resulting content was then transferred through a filter into a flow cytometry tube and analyzed by flow cytometry (Gallios, Beckman Coulter). Further, another test was conducted, as a control test, similarly to the manner described earlier, except that the ODN#3, ODN#2/SPG complex or ODN#3/SPG was not added. The other test was conducted similarly to the manner described earlier, except that the ODN#3 or ODN#3/SPG complex was not added.

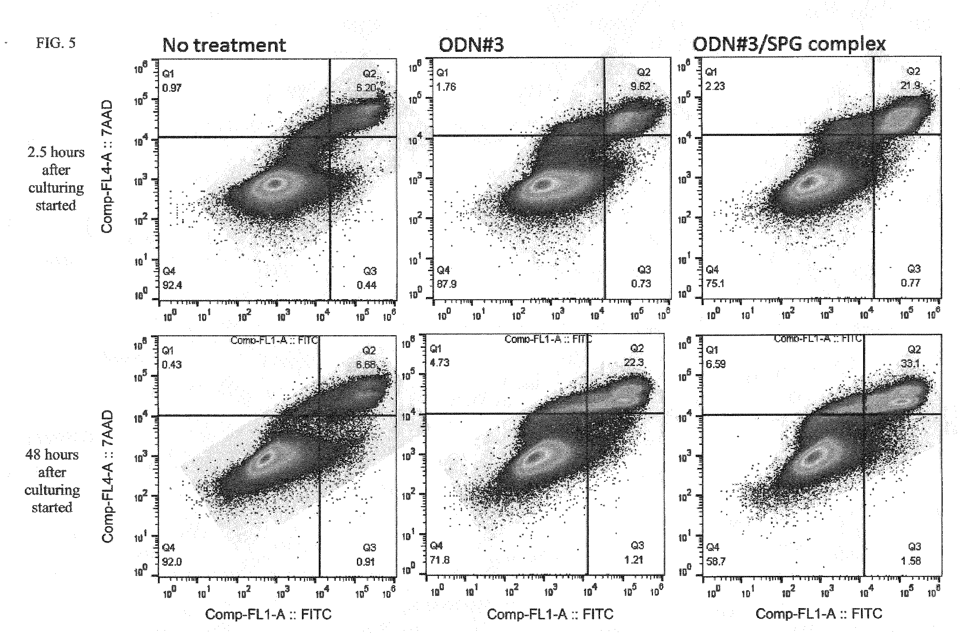

Fig. 5 shows the obtained result. According to this result, when the ODN#3/SPG complex was added, dead cells increased in short time after the culturing started, and the proportion of dead cells did not change after 48 hours passed when the growth of living cancer cells should be expected. This result demonstrates that the ODN#3/SPG complex excels in inhibiting the growth of cancer cells.

### Test 7: Evaluation on performance of inhibiting the adhesion activity of healthy pneumocytes

This test evaluated the performance of the ODN#3/SPG complex in inhibiting the adhesion activity of healthy cells. First, the whole cells were collected from lungs of two BAlb/c female mice (lung whole cells) to prepare a cell suspension (DMEM (FBS 10%)) containing 2 × 10⁵ cells/ml of the pneumocytes. Then, 100µl of the cell suspension was put in each well of a 96-well plate, and the SPG (final concentration: 5.8 µg/ml), ODN#3-dA(s)40 (final concentration: 0.5 µM or 1.0 µM), or ODN#3/SPG complex (final concentration: 0.5 µM or 10 µM) was further added to each well to culture the cells. The test materials were microscopically observed in 20 hours after the culturing started. Further, another test was conducted, as a control test, similarly to the manner described earlier, except that PBS was added instead of the SPG, ODN#3-dA(s)40 and ODN#3/SPG complex.

Microscopic observation of the cells adhered to the wells confirmed that the ODN#3/SPG complex did not inhibit the adhesion of healthy pneumocytes.

### Test 8: Evaluation on performance of inhibiting the adhesion activity of healthy lung fibroblasts

This test evaluated the performance of the ODN#3/SPG complex in inhibiting the adhesion activity of healthy cells. First, lung fibroblasts were collected from lungs of one BAlb/c female mouse with use of collagenase to prepare a cell suspension (DMEM (FBS 10%)) containing 3 × 10⁵ cells/ml of the pneumocytes. Then, 100µl of the cell suspension was put in each well of a 96-wellplate, and the SPG (final concentration: 6.3 µg/ml), ODN#3-dA(s)40 (final concentration: 0.5 µM or 1.0 µM), or ODN#3/SPG complex (final concentration: 0.5 µM or 10 µM) was further added to each well to culture the cells. The test materials were microscopically observed in 3 hours and 24 hours after the culturing started. Further, another test was conducted, as a control test, similarly to the manner described earlier, except that PBS was added instead of the SPG, ODN#3-dA(s)40 and ODN#3/SPG complex.

Microscopic observation of the cells adhered to the wells confirmed that the ODN#3/SPG complex did not inhibit the adhesion of healthy lung fibroblasts. The results of the tests 7 and 8 expressly teach that the ODN#3/SPG complex does not inhibit the adhesion activity of healthy cells but specifically inhibits the adhesion activity of cancer cells.

### Test 9: Ex-vivo evaluation on performance of inhibiting the engraftment of cancer cells

This test conducted an ex-vivo evaluation on performance of inhibiting engraftment of cancer cells using the ODN#3/SPG complex. First, a cell suspension containing Colon26-Luc (Colon-26; mouse cell line that expresses luciferase, JCRB1496) (RPMI (FBS 10%)) was put in a dish at the rate of 5 × 10⁶ cells/dish. Next, the ODN#3-dA(s)40 or ODN#3/SPG complex was added to the dish at the final concentration of 1 µM, and the cells were cultured for four hours at 37°C. The cultured cells were collected and suspended in PBS at the rate of 2 × 10⁶ cells/ml. Then, 500 µl of the obtained cell suspension was administered to BALB/c mice (female, 10-week old) through tail vein injection (i.v.), and the mice were raised. At day 5 and day 10 after the suspension was administered, lungs were isolated from the mice, and pneumocytes were subjected to a luciferase assay. Other tests were further conducted as control tests. One of the tests was conducted similarly to the manner described earlier, except that PBS was added instead of the ODN#3-dA(s)40 and ODN#3/SPG complex to culture the Colon26-Luc cells. In the other test, mice not administered with the cell suspension, as control subjects, were tested similarly to the manner described earlier.

The luciferase assay of the pneumocytes was performed according to the following steps. The mice lungs were cut into 4 mm-square pieces with straight scissors, and a lung unicellular suspension was prepared with use of the Tumor Dissociation Kit (MiltenyiBiotek) and the Gentle MACS Cell Dissociator (MiltenyiBiotek). Then, RPMI 1640 was added to the prepared lung unicellular suspension so as to amount in total to 350 µl. While the cell solution thus prepared was being cooled with ice, the cells were pulverized with a sonicator for 10 seconds repeatedly three times. Then, 350 µl of a OneGlo solution (Promega) was added to the obtained cell-pulverized solution and mixed upside-down 10 times, and the supernatant was collected by centrifugal separation (12,000 × g, 3 minutes). Then, 200µl of the supernatant was transferred into a 96-well white plate (3 wells/sample), and luminescence was immediately measured with a luminometer (GloMax, Promeg).

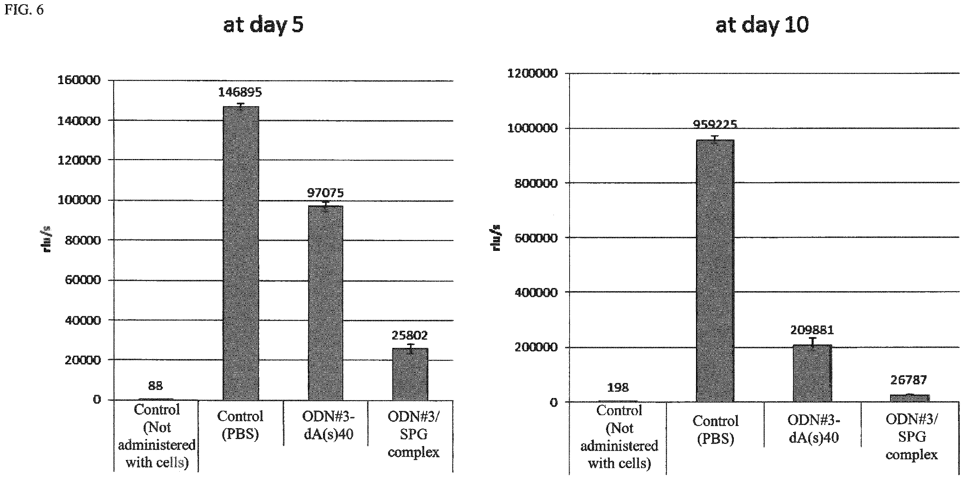

Fig. 6 shows the obtained result. When the cancer cells cultured in the presence of the ODN#3-dA(s)40 were transplanted in the mice lungs, the cancer cells were engrafted in the lungs. The cancer cells cultured in the presence of the ODN#3/SPG complex, on the other hand, were markedly inhibited from being engrafted in the lungs. This result demonstrates that the ODN#3/SPG complex excels in inhibiting the engraftment of cancer cells.

### Test 10: In-vivo evaluation on performance of inhibiting the engraftment of cancer cells

This test conducted an in-vivo evaluation on performance of inhibiting the engraftment of cancer cells using the ODN#3/SPG complex. First, 500 µl of a cell suspension containing 2 × 10⁶ cells/ml of Colon26-Luc (Colon-26; mouse cell line that expresses luciferase, JCRB1496) was administered to BALB/c mice (female, 10-week old) through tail vein injection (1 × 10⁶ cells/head). Subsequent to day 0 when the cell suspension was administered, the ODN#3-dA(s)40 or ODN#3/SPG complex was further administered to the mice at day 1, 3, 5, 7, and 9 at the rate of 10 µg/300 µl/head. At day 10, lungs were isolated from the mice, and pneumocytes were subjected to a luciferase assay in a manner similar to the test 9. Other tests were further conducted as control tests. One of the tests was conducted similarly to the manner described earlier, except that PBS was administered instead of the ODN#3-dA(s)40 and ODN#3/SPG complex. In the other test, mice not administered with the cell suspension, ODN#3-dA(s)40 or ODN#3/SPG complex, as control subjects, were tested similarly to the manner described earlier.

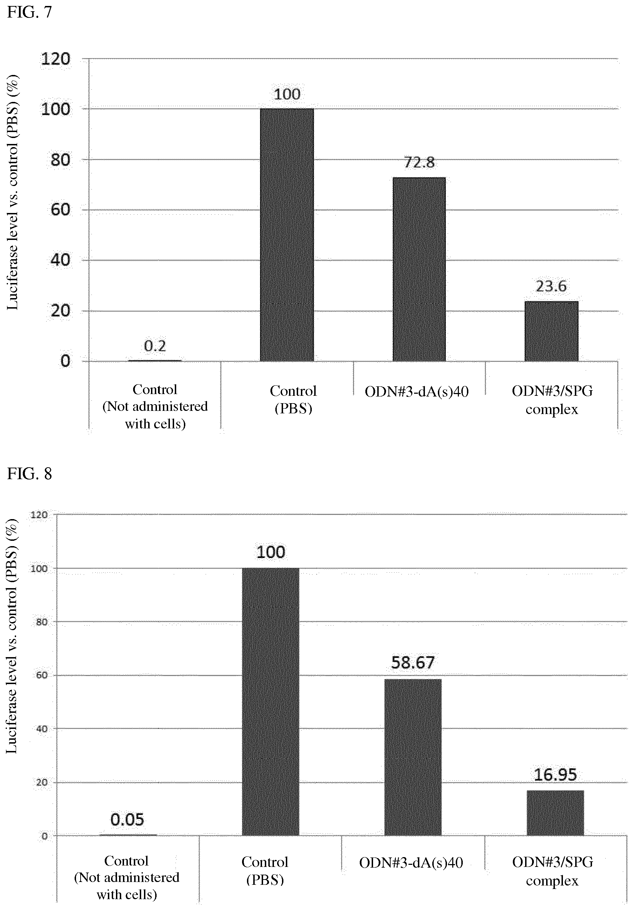

Fig. 7 shows the obtained result. This result demonstrates that the transplanted cancer cells were effectively inhibited from being engrafted into the lungs by administering the ODN#3/SPG complex.

### Test 11: In-vivo evaluation on performance of inhibiting the engraftment of cancer cells and immune-related effects in spleen

This test conducted an in-vivo evaluation on performance of inhibiting the engraftment of cancer cells and immune-related effects in spleen when the ODN#3/SPG complex was used. First, 500 µl of a cell suspension containing 2 × 10⁶ cells/ml of Colon26-Luc (Colon-26; mouse cell line that expresses luciferase, JCRB1496) was administered to BALB/c mice (female, 10-week old) through tail vein injection (1 × 10⁶ cells/head). Subsequent to day 0 when the cell suspension was administered, the ODN#3-dA(s)40 or ODN#3/SPG complex was further administered to the mice at day 5, 7, and 9 at the rate of 10 µg/300 µl/head. At day 10, lungs and spleens were isolated from the mice. The pneumocytes of the isolated lungs were subjected to a luciferase assay in a manner similar to the test 9. The isolated spleens were used to prepare a spleen-derived cell suspension. The cell suspension was dyed using F4/80:FITC, CCR7:PE, 7AAD, H-2Kd:APC and CD11c:PB and was also dyed using CD8:FITC, IgM:PE, 7AAD, H-2Kd:APC and CD80:PB, and was then analyzed by flow cytometry (Gallios, Beckman Coulter).

Other tests were further conducted as control tests. One of the tests was conducted similarly to the manner described earlier, except that PBS was administered instead of the ODN#3-dA(s)40 and ODN#3/SPG complex. In the other test, mice not administered with the cell suspension, ODN#3-dA(s)40 or ODN#3/SPG complex, as control subjects, were tested similarly to the manner described earlier.

Fig. 8 shows the luciferase assay result of the isolated lungs. This result also demonstrates that the transplanted cancer cells were effectively inhibited from being engrafted into the lungs by administering the ODN#3/SPG complex.

According to the flow cytometry analysis result of the isolated spleens, cell colonies that highly express MHC class I in the spleens more pronouncedly increased in the subjects administered with the ODN#3-dA(s)40 and ODN#3/SPG complex. The subjects administered with the ODN#3/SPG complex exhibited the tendency to inhibit CD8-positive cells, without inflammatory macrophage being activated. As compared with the control subjects and the subjects administered with the ODN#3-dA(s)40, cells that express CCR7; migration marker for cancer cells and native T cells, further decreased in the subjects administered with the ODN#3/SPG complex. This confirms that the performance of the ODN#3/SPG complex in inhibiting the engraftment of cancer cells does not rely on the CD8-positive cells being activated in the subjects' spleens.

### Test 12: In-vivo evaluation on performance of inhibiting the engraftment of cancer cells and immune-related effects in spleen

This test conducted an in-vivo evaluation on performance of inhibiting the engraftment of cancer cells and immune-related effects in spleen when the ODN#3/SPG complex was used. First, 500 µl of a cell suspension containing 2 × 10⁶ cells/ml of Colon26-Luc (Colon-26; mouse cell line that expresses luciferase, JCRB1496) was administered to BALB/c mice (female, 12-week old) through tail vein injection (1 × 10⁶ ells/head). Subsequent to day 0 when the cell suspension was administered, the ODN#3-dA(s)40 or ODN#3/SPG complex was further administered to the mice at day 1, 3 and 5 at the rate of 10 µg/300 µl/head. At day 6, lungs and spleens were isolated from the mice. The pneumocytes of the isolated lungs were subjected to a luciferase assay in a manner similar to the test 9. The isolated spleens were used to prepare a spleen-derived cell suspension. The cell suspension was dyed using Gr-1/FITC, DX5/PE, 7AAD, H-2Kd/APC and CD80/PB and was also dyed using CD40/FITC, CCR7/PE, 7AAD, H-2Kd/APC and CD80/PB, and was then analyzed by flow cytometry (Gallios, Beckman Coulter).

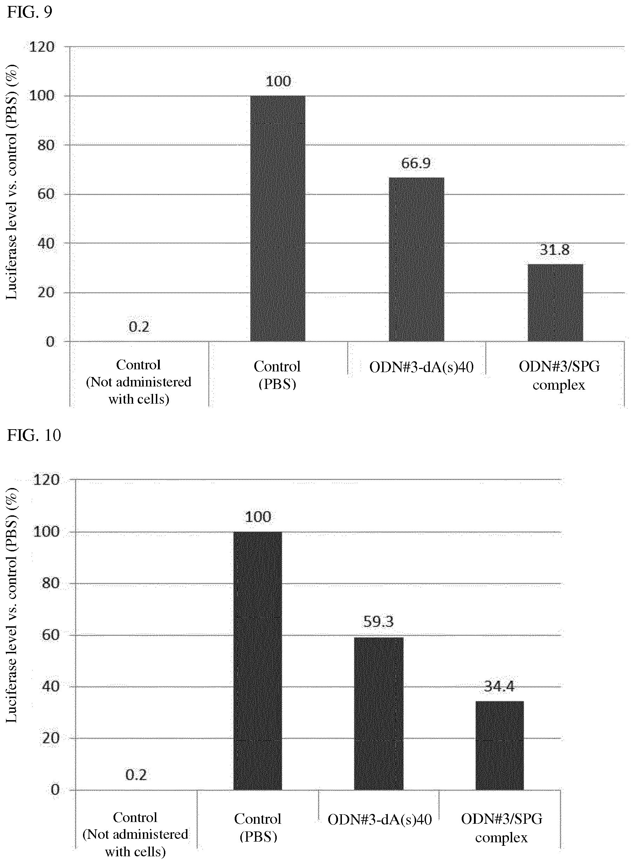

Fig. 9 shows the luciferase assay result of the isolated lungs. This result also demonstrates that the transplanted cancer cells were effectively inhibited from being engrafted into the lungs by administering the ODN#3/SPG complex.

The flow cytometry analysis result of the isolated spleens teaches that, as compared with control (PBS-administered) subjects, the ratio of NK cell colonies certainly decreased in the subjects administered with the ODN#3-dA(s)40 and ODN#3/SPG complex, however, the lowest ratio was marked in the subjects administered with the ODN#3/SPG complex. As for the ratio of mature granulocytes, there was substantially no difference among the control subjects, ODN#3-dA(s)40-administered subjects and ODN#3/SPG complex-administered subjects. As compared with control (PBS-administered) subjects, the ratio of cells that express CCR7; migration marker for native T cells and cancer metastasis, certainly decreased in the subjects administered with the ODN#3-dA(s)40 and ODN#3/SPG complex, however, more notably decreased in the subjects administered with the ODN#3/SPG complex. As for CD40 and CD80 that appears to be expressed in substantially the same cell colonies, the ratio of cells that express CD80 increased in the subjects administered with the ODN#3-dA(s)40, while the ratio of cells that express CD40 increased in the subjects administered with the ODN#3/SPG. These results provide no categorical indication of immune activation in spleens of the subjects administered with the ODN#3-dA(s)40 and ODN#3/SPG complex. This confirms that the performance of the ODN#3/SPG complex in inhibiting the engraftment of cancer cells is not associated with immune-related effects in the subjects' spleens.

### Test 13: In-vivo evaluation on performances of inhibiting the engraftment of cancer cells and immune-related effects in spleen

This test conducted an in-vivo evaluation on performance of inhibiting the engraftment of cancer cells and immune-related effects in spleen when the ODN#3/SPG complex was used. First, 500 µl of a cell suspension containing 2 × 10⁶ cells/ml of Colon26-Luc (Colon-26; mouse cell line that expresses luciferase, JCRB1496) was administered to BALB/c mice (female, 8-week old) through tail vein injection (1 × 10⁶ cells/head). Subsequent to day 0 when the cell suspension was administered, the ODN#3-dA(s)40 or ODN#3/SPG complex was further administered to the mice at day 1 at the rate of 10 µg/300 µl/head. At day 5, lungs and spleens were isolated from the mice. The pneumocytes of the isolated lungs were subjected to a luciferase assay in a manner similar to the test 9. The isolated spleens were used to prepare a spleen-derived cell suspension. The cell suspension was dyed using EpCAM/FITC, CD44/PE, 7AAD and CD11b/APC and was then analyzed by flow cytometry (Gallios, Beckman Coulter).

Fig. 10 shows the luciferase assay result of the isolated lungs. This result also demonstrates that the transplanted cancer cells were effectively inhibited from being engrafted into the lungs by administering the ODN#3/SPG complex.

As for the ratios of living cells and dead cells, the flow cytometry analysis result on the isolated spleens shows no distinct difference among the control subjects, ODN#3-dA(s)40-administered subjects and ODN#3/SPG complex-administered subjects. This result also shows that EpCAM (CD326); indicator of cell adhesion, cell growth and cell progression, decreased in the subjects administered with the ODN#3/SPG complex.

### Test 14 In-vivo evaluation on performances of inhibiting engraftment of cancer cells and immune-related effects in spleen

This test conducted an in-vivo evaluation on performance of inhibiting the engraftment of cancer cells and immune-related effects in spleen when the ODN#3/SPG complex was used. First, 500 µl of a cell suspension containing 2 × 10⁶ cells/ml of Colon26-Luc (Colon-26; mouse cell line that expresses luciferase, JCRB1496) was administered to BALB/c mice (female, 10-week old) through tail vein injection (1 × 10⁶ cells/head). Subsequent to day 0 when the cell suspension was administered, the ODN#3-dA(s)40 or ODN#3/SPG complex was further administered to the mice at day 0 at the rate of 10 µg/300 µl/head. At day 5, lungs and spleens were isolated from the mice. The pneumocytes of the isolated lungs were subjected to a luciferase assay in a manner similar to the test 9. The isolated spleens were used to prepare a spleen-derived cell suspension. The cell suspension was dyed using DX5/FITC, CXCR4/PE, 7AAD, NKG2D/APC and CD11c/PB and was also dyed using EpCAM/FITC, CD3/PE, 7AAD, CD8/APC and CD11c/PB, and was then analyzed by flow cytometry (Gallios, Beckman Coulter).

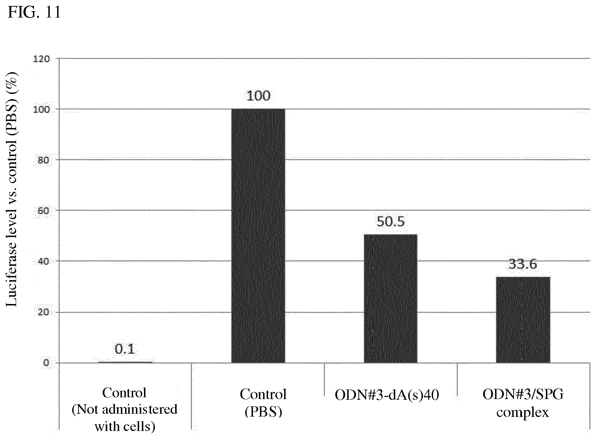

Fig. 11 shows the luciferase assay result of the isolated lungs. This result also demonstrates that the transplanted cancer cells were effectively inhibited from being engrafted into the lungs by administering the ODN#3/SPG complex.

The flow cytometry analysis result on the isolated spleens shows that, as compared with the control subjects, activation-induced NK cells were observed in the subjects administered with the ODN#3-dA(s)40 and in the subjects administered with the ODN#3/SPG complex. As compared with the control subjects and the subjects administered with the ODN#3-dA(s)40, cells that express CXCR4; metastasis marker for cancer cells, and cells that express EpiCAM; marker indicative of a state of adhesion and growth of cancer cells, both decreased, and activated NK cells were enhanced in the subjects administered with the ODN#3/SPG complex. The activation of T cells was not observed in the subjects administered with the ODN#3/SPG complex but was observed (increased ratio of CD8+ cells) in the subjects administered with the ODN#3-dA(s)40. This confirms that the inhibitory action of the ODN#3-SPG complex against the engraftment of cancer cells is a result mediated by successful inhibition of the adhesion and growth of cancer cells, regardless of activation of T cells.

## Claims

1. An agent for inhibiting adhesion activity of cancer cells comprising a complex of nucleic acid molecules and a polysaccharide, wherein
the nucleic acid molecule includes an oligodeoxynucleotide in which phosphodiester bonds are phosphorothioated, and
the polysaccharide is a polysaccharide having a β-1,3-glucan skeleton.

2. An agent for inhibiting proliferation of cancer cells comprising a complex of nucleic acid molecules and a polysaccharide, wherein
the nucleic acid molecule includes an oligodeoxynucleotide in which phosphodiester bonds are phosphorothioated, and
the polysaccharide is a polysaccharide having a β-1,3-glucan skeleton.

3. An agent for inhibiting engraftment of cancer cells comprising a complex of nucleic acid molecules and a polysaccharide, wherein
the nucleic acid molecule includes an oligodeoxynucleotide in which phosphodiester bonds are phosphorothioated, and
the polysaccharide is a polysaccharide having a β-1,3-glucan skeleton.

4. An agent for inhibiting cancer metastasis comprising a complex of nucleic acid molecules and a polysaccharide, wherein
the nucleic acid molecule includes an oligodeoxynucleotide in which phosphodiester bonds are phosphorothioated, and
the polysaccharide is a polysaccharide having a β-1,3-glucan skeleton.

5. An agent for treating cancer comprising a complex of nucleic acid molecules and a polysaccharide, wherein
the nucleic acid molecule includes an oligodeoxynucleotide in which phosphodiester bonds are phosphorothioated (except for CpG oligodeoxynucleotides), and
the polysaccharide is a polysaccharide having a β-1,3-glucan skeleton.

6. The agent according to any one of claims 1 to 5, wherein the polysaccharide is schizophyllan.

7. The agent according to any one of claims 1 to 6, wherein polydeoxyadenine is added to the oligodeoxynucleotide.

8. The agent according to claim 7, wherein the polydeoxyadenine includes 10 to 100 deoxyadenines.

9. The agent according to any one of claims 1 to 8, wherein the oligodeoxynucleotide has 5 to 40 bases.

10. A method for inhibiting adhesion activity of cancer cells, the method comprising a step of administering a complex of nucleic acid molecules and a polysaccharide to a person in whom the adhesion activity of cancer cells needs to be inhibited, wherein
the nucleic acid molecule includes an oligodeoxynucleotide in which phosphodiester bonds are phosphorothioated, and
the polysaccharide is a polysaccharide having a β-1,3-glucan skeleton.

11. A method for inhibiting proliferation of cancer cells, the method comprising a step of administering a complex of nucleic acid molecules and a polysaccharide to a person in whom the growth of cancer cells needs to be inhibited, wherein
the nucleic acid molecule includes an oligodeoxynucleotide in which phosphodiester bonds are phosphorothioated, and
the polysaccharide is a polysaccharide having a β-1,3-glucan skeleton.

12. A method for inhibiting engraftment of cancer cells, the method comprising a step of administering a complex of nucleic acid molecules and a polysaccharide to a person in whom the engraftment of cancer cells needs to be inhibited, wherein
the nucleic acid molecule includes an oligodeoxynucleotide in which phosphodiester bonds are phosphorothioated, and
the polysaccharide is a polysaccharide having a β-1,3-glucan skeleton.

13. A method for inhibiting cancer metastasis, the method comprising a step of administering a complex of nucleic acid molecules and a polysaccharide to a person in whom the cancer metastasis needs to be inhibited, wherein
the nucleic acid molecule includes an oligodeoxynucleotide in which phosphodiester bonds are phosphorothioated, and
the polysaccharide is a polysaccharide having a β-1,3-glucan skeleton.

14. A method for treating cancer comprising a step of administering a complex of nucleic acid molecules and a polysaccharide to a cancer patient, wherein
the nucleic acid molecule includes an oligodeoxynucleotide in which phosphodiester bonds are phosphorothioated (except for CpG oligodeoxynucleotides), and
the polysaccharide is a polysaccharide having a β-1,3-glucan skeleton.

15. Use of a complex of nucleic acid molecules and a polysaccharide for the manufacture of an agent for inhibiting adhesion activity of cancer cells, wherein
the nucleic acid molecule includes an oligodeoxynucleotide in which phosphodiester bonds are phosphorothioated, and
the polysaccharide is a polysaccharide having a β-1,3-glucan skeleton.

16. Use of a complex of nucleic acid molecules and a polysaccharide for the manufacture of an agent for inhibiting proliferation of cancer cells, wherein
the nucleic acid molecule includes an oligodeoxynucleotide in which phosphodiester bonds are phosphorothioated, and
the polysaccharide is a polysaccharide having a β-1,3-glucan skeleton.

17. Use of a complex of nucleic acid molecules and a polysaccharide for the manufacture of an agent for inhibiting engraftment of cancer cells, wherein
the nucleic acid molecule includes an oligodeoxynucleotide in which phosphodiester bonds are phosphorothioated, and
the polysaccharide is a polysaccharide having a β-1,3-glucan skeleton.

18. Use of a complex of nucleic acid molecules and a polysaccharide for the manufacture of an agent for inhibiting cancer metastasis, wherein
the nucleic acid molecule includes an oligodeoxynucleotide in which phosphodiester bonds are phosphorothioated, and
the polysaccharide is a polysaccharide having a β-1,3-glucan skeleton.

19. Use of a complex of nucleic acid molecules and a polysaccharide for the manufacture of an agent for an agent for treating cancer, wherein
the nucleic acid molecule includes an oligodeoxynucleotide in which phosphodiester bonds are phosphorothioated (except for CpG oligodeoxynucleotides), and
the polysaccharide is a polysaccharide having a β-1,3-glucan skeleton.


### SEQUENCE LISTING

<110> NapaJen Pharma, Inc.
<120> Agent of inhibiting adhesive activity of cancer cell
<130> 18027WO
<150> JP2017-057975 <151> 2017-03-23
<160> 8
<170> PatentIn version 3.5
<210> 1 <211> 61 <212> DNA <213> Artificial Sequence
<220>
   <223> ODN#1-dA(S)40
<400> 1
   agttccattc tcccatagcc aaaaaaaaaa aaaaaaaaaa aaaaaaaaaa aaaaaaaaaa 60
   a 61
<210> 2 <211> 61 <212> DNA <213> Artificial Sequence
<220>
   <223> ODN#2-dA(S)40
<400> 2
   tggctatggg agaatggaac taaaaaaaaa aaaaaaaaaa aaaaaaaaaa aaaaaaaaaa 60
   a 61
<210> 3 <211> 60 <212> DNA <213> Artificial Sequence
<220>
   <223> ODN#3-dA(s)40
<400> 3
   atcgactctc gagcgttctc aaaaaaaaaa aaaaaaaaaa aaaaaaaaaa aaaaaaaaaa 60
<210> 4 <211> 60 <212> DNA <213> Artificial Sequence
<220>
   <223> ODN#4-dA(s)40
<400> 4
   atggactctg gagggttctc aaaaaaaaaa aaaaaaaaaa aaaaaaaaaa aaaaaaaaaa 60
<210> 5
   <211> 21
   <212> DNA
   <213> Artificial Sequence
<220>
   <223> ODN#1
<400> 5
   agttccattc tcccatagcc a 21
<210> 6
   <211> 21
   <212> DNA
   <213> Artificial Sequence
<220>
   <223> ODN#2
<400> 6
   tggctatggg agaatggaac t 21
<210> 7
   <211> 20
   <212> DNA
   <213> Artificial Sequence
<220>
   <223> ODN#3
<400> 7
   atcgactctc gagcgttctc 20
<210> 8
   <211> 20
   <212> DNA
   <213> Artificial Sequence
<220>
   <223> ODN#4
<400> 8
   atggactctg gagggttctc 20